(19) Europäisches Patentamt / European Patent Office / Office européen des brevets

(11) **EP 4 170 035 A1**

(12) **EUROPEAN PATENT APPLICATION**
published in accordance with Art. 153(4) EPC

(43) Date of publication:
**26.04.2023 Bulletin 2023/17**

(21) Application number: **20942241.9**

(22) Date of filing: **29.07.2020**

(51) International Patent Classification (IPC):
*C12N 15/60* (2006.01)     *A61K 35/18* (2015.01)

(52) Cooperative Patent Classification (CPC):
**A61K 35/18; A61K 38/51; A61K 48/00;
A61P 35/00; C12N 9/88; C12N 15/65; C12N 15/86;
C12N 15/867**

(86) International application number:
**PCT/CN2020/105381**

(87) International publication number:
**WO 2021/258492 (30.12.2021 Gazette 2021/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.06.2020 CN 202010591468**

(71) Applicant: **Guangzhou Sinogen Pharmaceutical
Co., Ltd
Guangzhou, Guangdong 510530 (CN)**

(72) Inventors:
• **ZHAO, Allan Zijian
Guangzhou, Guangdong 510006 (CN)**

• **LI, Fanghong
Guangzhou, Guangdong 510006 (CN)**
• **LI, Yuyu
Guangzhou, Guangdong 510006 (CN)**
• **ZHOU, Sujin
Guangzhou, Guangdong 510006 (CN)**
• **ZHAO, Zhenggang
Guangzhou, Guangdong 510006 (CN)**

(74) Representative: **Clements, Andrew Russell Niel
Schlich
9 St Catherine's Road
Littlehampton, West Sussex BN17 5HS (GB)**

Remarks:
The complete document including Reference
Table(s) and the Sequence Listing(s) can be
downloaded from the EPO website

(54) **APPLICATION OF METHIONINASE GENE THERAPY IN TREATMENT OF MALIGNANT TUMOR**

(57) Disclosed is an application of methioninase gene therapy in the treatment of a malignant tumor. In the gene therapy, a virus is used as a vector to insert an exogenous methioninase gene to constitute a methioninase expression system inside a tumor, thus providing an endogenous mechanism for further consumption of methionine. In vitro cytology experiments indicate that the methioninase gene therapy significantly reduces the level of intracellular methionine, effectively inhibits the proliferation of tumor cells, and can be used in an application in preparing a drug for the targeted treatment of a malignant tumor.

**Vector Map**

Figure 1

EP 4 170 035 A1

**Description**

**TECHNICAL FIELD**

The present invention relates to the fields of tumor treatment and gene therapy in medical technology field, in particular to use of methioninase gene therapy in the targeting treatment of malignant tumors.

**BACKGROUND**

Malignant tumor is a common disease that seriously threatens human life and health. According to the latest statistics in 2015, the mortality rate of malignant tumor accounts for 23.91% of all deaths of residents. In the past 10 years, the incidence of malignant tumors in China has an annual growth rate of 3.9%, and the mortality rate has an annual growth rate of 2.5%. That is, more than 10000 people are diagnosed with cancer every day and 7.5 people are diagnosed with cancer every minute. Medical expenses caused by malignant tumors exceed 220 billion, and the situation of prevention and control is severe. At present, there are many means and methods to treat malignant tumors, such as surgery, chemical drug therapy, radiotherapy, etc. These treatment methods have a certain effect on malignant tumors, but always have serious adverse reactions, limited efficacy and are easy to develop drug resistance, etc. Therefore, the key is to seek new targeting and precision medicine such as gene therapy, for the treatment of malignant tumors. Due to the rapid growth of tumor cells, the demand for nutrients and metabolism are different from those of normal undifferentiated cells. In order to maintain continuous proliferation, tumor cells have to adjust their metabolism and nutrient acquisition methods. A large number of basic and clinical trials have shown that new drugs can be developed for effectively inhibiting tumor growth by targeting the metabolism of tumor dependent amino acids and derivatives thereof, such as methionine, glutamine, glutamate, arginine, tryptophan, etc. Therefore, targeting metabolic regulation treatment is a new direction of tumor treatment.

Methionine dependence is a common feature of most tumor cells, such as breast cancer, prostate cancer, lung cancer, colon cancer, kidney cancer, bladder cancer, melanoma, glioma, etc., whereas normal cells do not exhibit methionine dependence and more serious malignant tumors show increased methionine dependence. Several experiments in vivo and vitro successively confirmed that direct consumption of a methionine deficient diet could delay tumor cell proliferation. However, long-term deficiency or insufficiency of dietary methionine will cause malnutrition and metabolic disorders, and can even exacerbate carcinogenesis by chronically DNA hypomethylating. Therefore, reducing methionine in vivo through the specific decomposition of methionine by methioninase MEGL can more effectively inhibit tumor cell growth or eliminate tumor cells. However, mammals do not express methioninase on their own, and exogenous giving has certain side effects and always evokes an immune reaction in the body. Therefore, exploring endogenously expressed methioninase is a better choice. The purpose of cancer gene therapy is to introduce therapeutic genes into tumor cells. These therapeutic genes introduced into target cells can correct the mutated gene, inhibit active oncogenes, or produce other properties on the cell, etc. Suitable exogenous therapeutic genes comprise, but are not limited to, immunotherapeutic genes, antiangiogenic genes, chemoprotective genes, and "suicide" genes, and they can be introduced into cells by utilizing modified viral vectors or nonviral methods (comprising electroporation, gene gun and lipid or polymer coating). Requirements for an optimal viral vector comprise the efficient ability to find a specific target cell and express the viral genome in the target cell. All these properties of viral vectors have been developed in the past few decades and extensively studied in biomedicine such as retrovirus, adenoviruses, adeno-associated viruses, and oncolytic viral vectors, etc.

Methionine belongs to the essential amino acid and, catalyzed by methionine adenosyltransferase, generates S-adenosylmethionine (SAM). SAM, also known as active methionine, is the most important direct methyl donor in the body and participates in the catalytic reaction of methyltransfer of DNA, proteins and other different substances in the body. Histone methyltransferase EZH2 is the active component of histone methyltransferase in polycomb repressive complex 2 (PRC2). It is involved in chromatin condensation by adding the active methyl group of SAM to histone lysine 27 (H3K27) of histone 3, thereby inhibiting the transcription of related genes (such as anti-oncogene). Studies found that EZH2 and histone H3K27 methylation are closely related to cancers. EZH2 was first found to have high expression in lymphomas, metastatic prostate and breast cancers, and to be associated with breast cancer invasion. In addition, EZH2 is overexpressed in many human malignancies, such as lung cancer, lymphoma, leukemia, pancreatic cancer, cervical cancer, intestinal cancer, liver cancer, gastric cancer, melanoma, kidney cancer and bladder cancer, and its expression level is significantly elevated in metastasized tumors, which is closely associated with poor prognosis of cancer patients. Preclinical models of drugs for targeting EZH2 show their ability to inhibit the progression of brain cancer and prostate cancer. Thus, EZH2 could serve as a potential drug target for treating cancer metastasis by reducing EZH2 expression and activity, thereby reducing the methylation of histones and enhancing the expression of anti-oncogene.

**SUMMARY OF THE INVENTION**

In the present applicantion, viral vectors were constructed by inserting exogenous MEGL gene to express methioninase. The vectors inhibit the growth and metastasis of malignant tumors by inhibiting the expression and activity of methyltransferase EZH2.

In one aspect, the present application provides a viral vector, wherein an exogenous MEGL gene is inserted therein.

Further, the exogenous MEGL gene is a methionine $\gamma$-lyase gene (GenBank accession No. l4313.1). Further, the vector the vector uses EF1A promoter.

Further, the vector carries a mcherry fluorescent protein.

Further, the exogenous MEGL gene consists of a sequence of SEQ ID No.1.

The vector is constructed by following steps of: subcloning a MEGL gene into a plasmid to obtain a MEGL expression plasmid; transfecting the MEGL expression plasmid and a helper plasmid into 293T cells; and collecting a supernatant, concentrating and purifying to obtain the viral vector. Further, the MEGL expression plasmid and the helper plasmid are co-transfected into the 293T cells, and the helper plasmid is viral packaging helper plasmid.

Further, the MEGL expression plasmid carries a mCherry red fluorescent protein.

The vector is constructed by following steps of:

(a) constructing an entry vector using BP reaction, comprising: mixing a Gateway expression vector having a target gene attB1- MEGL - attB2 sequence with a donor vector having attP1- ccdB - attP2 sequence; adding a BP Clonase enzyme mixture containing Int and IHF, keeping at 25 °C for 1h, and treating with a protease K at 37 °C for 10min to generate an entry vector having target gene MEGL and an expression vector having a suicide gene; transforming the entry vector into Escherichia coli Stbl3, and idendifying positive clones and performing sequencing validation;

(b) constructing a destination vector having two recombination sites attR1 and attR2 downstream of an expression regulatory element thereof, each of the recombination sites being 125 bp in length; and having a ccdB suicide gene between the attR1 and attR2; and

(c) constructing a final expression vector via LR reaction, comprising: mixing the entry vector and the destination vector, adding a LR Clonase enzyme mixture containing recombinant factors including Int, IHF and Xis, keeping at 25 °C overnight, performing transformation by treating with a proteinase K at 37 °C for 10 minutes to generate a fusion plasmid, attL1 sequence and the attR1 sequence being recombined, the fusion plasmid being decomposed into two new plasmids, obtaining a final expression vector of destination vector having the target gene; transforming the final expression vector into Escherichia coli Stbl3, and idendifying positive clone plasmid and performing sequencing validation.

Further, the viral vector is a lentivirus vector.

In another aspect, the present application provides use of the above viral vector in the manufacture of a medicament for treating malignant tumors.

Further, the medicament directly kills tumor cells.

Further, the malignant tumor is glioma.

In another aspect, the present application provides use of the above viral vector in the manufacture of histone methyltransferase EZH2 inhibitor.

In another aspect, the present application provides a method for treating malignant tumors, comprising administering the above viral vector or the viral vector constructed by the method above to subjects in need.

Further, the malignant tumor is glioma.

The viral vector is selected from the group consisting of lentivirus vector, retrovirus vector, adenovirus vector and other viral vectors known or under development in the art, and preferably is lentivirus vector.

The malignant tumors comprise but are not limited to glioma, breast cancer, prostate cancer, pancreatic cancer, liver cancer, colon cancer, rectal cancer, esophageal cancer, laryngeal cancer, leukemia, lymphatic cancer, melanoma, uterine cancer, ovarian cancer, skin cancer, bronchial cancer, bronchiolar cancer, urethral cancer, renal cancer, oral cancer, vaginal cancer, bile duct cancer, bladder cancer or nasopharyngeal cancer. The above medicaments or inhibitors can be administered via a variety of ways, comprising but not limited to: oral administration, local administration, injection (comprising but is not limitied to intravenous, peritoneal, subcutaneous, muscular, intratumoral, spinal administration), etc.

Compared with the prior art, the present invention has the following advantages:
The present invention provides a virus system for expressing methioninase. The virus system can inhibit the expression and activity of methyltransferase EZH2, thereby inhibiting the growth and metastasis of malignant tumors.

Compared with retroviruses and recombinant adenoviruses, the virus expression system has many the advantages. For example, the virus expression system can harbor large exogenous gene segments, allow stable and long-term gene expression, have high transfection efficiency, and not cause cellular immune response. At the same time, mCherry fluorescent protein has less cytotoxicity and is conducive to the observation of cell infection.

**BRIEF DESCRIPTION OF THE DRAWINGS**

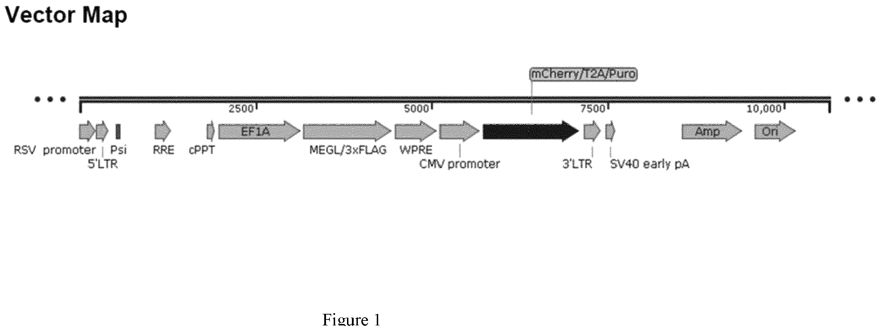

Figure 1 shows the construction of MEGL overexpression viral vector.

Figure 2 shows images of MEGL overexpression virus infected glioma cells captured with a fluorescence microscope.

Figure 3 shows results of PCR and Western Blot identification of MEGL overexpression virus infected target tumor cells. The upper image shows al time PCR results, and the lower images show stern Blot results. Group V: no-load virus control group; Group M: MEGL overexpression virus group.

Figure 4 shows LC-MS results showing a decrease in intracellular methionine level after infection with MEGL overexpression virus. Group V: no-load virus control group; Group M: MEGL overexpression virus group.

Figure 5 shows inhibition of the proliferation of glioma cells by MEGL overexpression virus. Group V: no-load virus control group; Group M: MEGL overexpression virus group. All data are expressed as mean $\pm$ standard deviation. (n=3), * P<0.05, ** P<0.01, *** P<0.001, all compared with group V.

Figure 6 shows inhibition of the expression of tumor cells EZH2 by MEGL overexpression virus. The upper one shows the down-regulation of EZH2 gene expression detected by 1 time PCR, and the lower ones show the down-regulation of EZH2 protein expression detected by Southern Blot. Group V: no-load virus control group; Group M: MEGL overexpression virus group. All data are expressed as mean $\pm$ standard deviation. (n=3), * P<0.05, ** P<0.01, *** P<0.001, all compared with group V.

**DETAILED DESCRIPTION OF THE EMBODIMENTS**

The present application will be further illustrated with reference to following embodiments. However, these embodiments are only for illustrating, rather than limitations to the present invention detailed in the claims.

**Example 1: Construction of expression vector having MEGL**

MEGL/3xFLAG of following sequence (SEQ ID NO. 1) is used:

1 ATGCGC GACTCC CATAAC AACACC GGTTTT TCCACA CGGGCC ATTCAC CACGGC TACGAC

61 CCGCTT TCCCAC GGTGGT GCCTTG GTGCCA CCGGTG TACCAG ACCGCG ACCTAT GCCTTC

121 CCGACT GTCGAA TACGGC GCTGCG TGCTTC GCCGGG GAGGAG GCGGGG CACTTC TACAGC

181 CGCATC TCCAAC CCCACC CTGGCC TTGCTC GAGCAA CGCATG GCCTCG TTGGAG GGTGGT

241 GAGGCG GGATTG GCGCTG GCGTCG GGGATG GGAGCC ATTACT TCGACC CTCTGG ACCCTG

301 CTGCGG CCTGGT GATGAG CTGATC GTGGGG CGCACC TTGTAT GGCTGC ACCTTT GCGTTC

361 CTGCAC CATGGC ATTGGC GAGTTC GGGGTC AAGATC CACCAT GTCGAC CTTAAC GATGCC

421 AAGGCC CTGAAA GCGGCG ATCAAC AGCAAA ACGCGG ATGATC TACTTC GAAACA CCGGCC

481 AACCCC AACATG CAACTG GTGGAT ATAGCG GCGGTC GTCGAG GCAGTG CGGGGG AGTGAT

541 GTGCTT GTGGTG GTCGAC AACACC TACTGC ACGCCC TACCTG CAGCGG CCACTG GAACTG

601 GGGGCA GACCTG GTGGTG CATTCG GCAACC AAGTAC CTCAGT GGCCAT GGCGAC ATCACT

661 GCGGGC CTGGTG GTGGGG CGCAAG GCTTTG GTCGAC CGCATT CGGCTG GAAGGG CTGAAA

721 GACATG ACCGGG GCAGCC TTGTCA CCGCAT GACGCT GCGTTG TTGATG CGCGGC ATCAAG

781 ACCCTG GCGCTG CGCATG GACCGG CATTGC GCCAAC GCCCTG GAGGTC GCGCAG TTCCTG

841 GCCGGG CAGCCC CAGGTG GAGCTG ATCCAC TACCCG GCTTG CCGTCG TTTGCC CAGTAC

901 GAACTG GCACAG CGGCAG ATGCGT TTGCCG GCCGGG ATGATT GCCTTT GAGCTC AAGGGC

961 GGTATC GAGGCC GGGCGC GGCTTC ATGAAT GCCCTG CAGCTT TTTGCC CGTGCG GTGAGC

1021 CTGGGG GATGCC GAGTCG CTGGCA CAGCAC CCGGCG AGCATG ACGCAC TCCAGT TACACG

1081 CCACAA GAGCGG GCGCAT CACGGG ATATCA GAGGGG CTGGTG AGGTTG TCAGTG GGGCTG

1141 GAGGAT GTGGAG GACCTG CTGGCA GATATC GAGTTG GCGTTG GAGGCG TGTGCA GACTAC

1201 AAAGAC CATGAC GGTGAT TATAAA GATCAT GATATC GATTAC AAGGAT GACGAT GACAAG

1261 TGA

The viral vector is purchased from Cyagen Biology. The expression vector is mainly constructed using Gateway cloning technology. Gateway technology comprises two reactions, BP reaction and LR reaction. The BP reaction uses a recombination reaction between an attB DNA segment or expression clone and an attP donor vector to create an entry clone. The LR reaction is a recombination reaction between an attL entry clone and an attR destination vector. The details are described as follows:

1) Construction of the entry vector via BP reaction: Gateway expression vector having a target gene (attB1- MEGL-attB2 sequence) is mixed with a donor vector having attP1- ccdB (suicide gene) - attP2 sequence, and a BP Clonase enzyme mixture containing Int and IHF is added thereto. The resulted is kept at 25 °C for 1h and then treated with a protease K at 37 °C for 10min, so that recombination of attP and attB sequences occurs, generating an entry vector having target gene (MEGL) and an expression vector having suicide gene. The entry vector is transformed into Escherichia coli Stbl3, and positive clones are identified and sequencing validation is performed.

2) The destination vector, for matching with the Gateway system, has two recombination sites attR1 and attR2 downstream of an expression regulatory element thereof. Each of the recombination sites is 125 bp in length; and there is a suicide gene ccdB between the attR1 and attR2.

3) Construction of final expression vector via LR reaction: Two plasmids, i.e. the entry vector and the destination vector, are mixed, and a LR Clonase enzyme mixture containing recombinant factors including Int, IHF and Xis is added thereto. The resulted is kept at 25 °C overnight and then treated with a proteinase K at 37 °C for 10 minutes to perform transformation, so that the attR2 and attL2 sequences are recombined to generate a fusion plasmid. The attL 1 sequence is recombined with the attR1 sequence, and the fusion plasmid is decomposed into two new plasmids, obtaining a final expression vector of destination vector having the target gene. The target product is transformed into Escherichia coli Stbl3, and positive clone plasmids are identified and sequencing validation is performed.

**Example 2: Preparation of MEGL overexpression virus (using lentivirus as an example)**

1) Virus packaging: The day before transfection, 293T cells are seeded in a culture dish. The number of seeded cells should be as such to allow cells growth to reach 90% - 95% confluency on the day of transfection. On the day of transfection, culture medium is removed from 293T cells, and 10 mL (10 cm culture dish) of a culture medium for virus packaging is added thereto. Calcium phosphate-DNA precipitation is prepared as follows: A) Calcium-DNA mixture: $CaCl_2$ is added into a 5mL sterile EP tube, then an auxiliary plasmid and target gene plasmid are added thereto and mixed well. B) The centrifuge tube containing the calcium-DNA mixture is placed on a vortex oscillator to vortex the liquid, and then 2×HBS is added dropwise. After the dropping is completed, vortex for a few seconds and let stand for 5 minutes. The calcium phosphate-DNA suspension is poured into the culture medium of the above cells and mixed well gently, and placed in a 37 °C, 5% $CO_2$ saturated humidity incubator for culturing; After 4-6 hours of transfection, the culture medium is removed, and 10 mL of a culture medium for virus packaging is added. Then culture is continued in a 37 °C, 5% $CO_2$ saturated humidity incubator.

2) Collecting and concentrating virus: 48h after transfection, the culture medium containing virus is collected into a 50mL centrifuge tube; The virus supernatant was centrifuged at low speed to remove cell fragments. The resulted supernatant is collected and filtered with a 0.45 μM small filter, and the filtrate is collected. PEG6000 and NaCl solution are added thereto in an amount corresponding to the volume of filtrate and mixed well, placed at 4 °C overnight for precipitation, and centrifuged the next day at 4 °C, 1500×g for 30 minutes. The supernatant is removed; The virus precipitate is dissolved with HBSS, and fully pipetted up and down to prepare a single virus suspension which is then sub packaged into cryogenic vials.

3) Identifying virus titer via Real time PCR: The day before the virus infection, 293T are seeded in a 6-well plate, five plates for each well, and $5 \times 10^5$ cells/well; After 24 hours of the seeding, cells from two wells are counted with a hemocytometer to determine the actual number of cells at the time of infection, which is recorded as N. The medium in other culture plates are discarded and replaced with fresh culture medium to a final concentration of 5 μg/mL polybrene. The concentrated virus is diluted 200 times with medium, that is, 1μL virus is added to 199μL medium. 0.5 μL, 5 μL and 5 μL diluted virus are added to three culture wells, respectively; 20 hours after infection, culture supernatant is removed and replaced with 500 μL fresh culture medium having DNaseI. Digest at 37 °C for 15 minutes to remove the residual plasmid DNA. The cells are then cultured in 2mL normal medium for 48 hours, digested with 0.5 mL 0.25% trypsin-EDTA solution, and collected by centrifugation. Extract genomic DNA according to the instructions of DNeasy kit and conduct real time fluorescent PCR amplification. Titer (integration units per mL, IU/mL) is calculated according to formula as follows:

$$IU/mL = (C \times N \times D \times 1000) / V$$

wherein: C = average copy number of virus integrated per genome; N = number of cells at the time of infection (approximately $1 \times 10^6$) D=dilution ratio of viral vector; V = volume of diluted virus added.

**Example 3: The anti-tumor effect of MEGL overexpressing virus.**

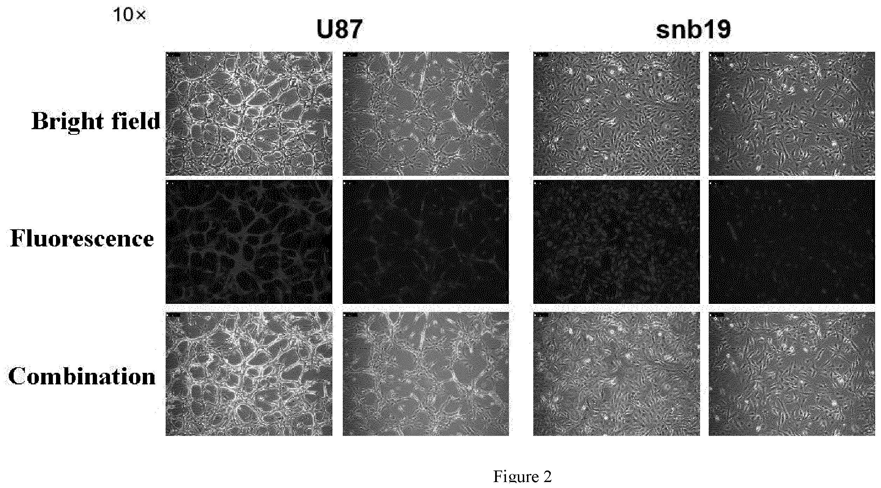

1. Glioma cells U87 and snb19 in logarithmic growth phase are inoculated into a 6-well plate. When the cells are grown to a density of about 30% - 50%, a control no-load viral vector (hereinafter defined as Group V) and MEGL overexpression lentivirus (hereinafter defined as Group M) are added according to MOI=10, and Polyprene was added as an aid to a final concentration of 5μg/mL. After 24h, replace with normal medium and continue culture for 48h. Screen with Puromycin having a final concentration of 2μg/mL. Fluorescence intensity and proportion of cells are observed under a fluorescence microscope every 24 hours. The screen is successful when the number of red fluorescence cells exceeds 95%. Cell screening results are shown in Figure 2.

2. Real time PCR and Western Blot for identifying the expression of methioninase in cells infected with MEGL virus.

Total RNA is extracted from well-grown cells after virus infection using Trizol method. 1μg of the total RNA template is reverse transcribed into cDNA in vitro. With cDNA as template, amplification is completed after 30 cycles of pre-denaturation (94°C, 2min), denaturation (94°C, 30s), annealing (55°C, 30s), elongation (72°C, lmin), and a final elongation (72°C, 10min). The amplification products are analyzed by agarose gel electrophoresis, and images are captured using the gel imaging system.

Wherein, the MEGL primer sequence is as follows:

MEGL-F: CACTTCTACAGCCGCATCTCCAAC
MEGL-R: GACCACCACAAGCACATCACTCC

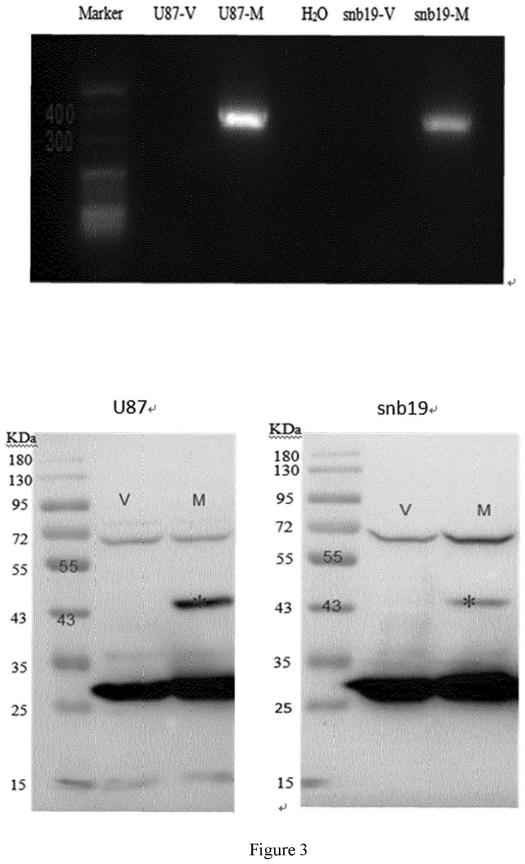

The results are shown in Figure 3A. The PCR results show that specific MEGL target gene segment (387bp) is detected at 300-400bp in group M, which indicates that the glioma is successfully infected by MEGL overexpression virus. 200 $\mu$L RIPA lysis and 2 $\mu$L PMSF are added to well-grown cells after virus infection. Cells are scraped off with a clean cell scraper and transferred into a new 1.5 ml EP tube. The cells are lysed on ice for 10 min, broken up by sonication for 3s with 3s interval for a total of 30s, and then centrifuged at 12000 rpm at 4 °C for 15 min. The supernatant is collected into another clean EP tube, and stored at -20 °C. The total protein concentration is detected by BCA method. MEGL is detected by Western Blot, using Tubulin as an internal control. As shown in Figure 3B, specific bands are detected in group M at about 43KD (* represents methioninase protein band), further indicating successful infection with MEGL overexpression virus.

**3. Identifying the methioninase activity of cells infected with MEGL overexpression virus. Intracellular methionine levels in malignant tumor cells infected by MEGL overexpression virus are determined with Liquid chromatography mass spectrometry (LC-MS).**

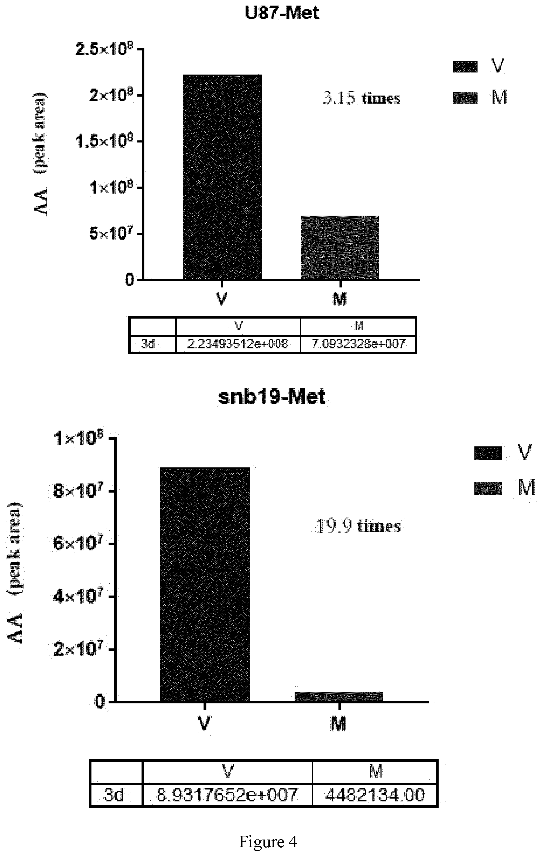

Virus infected glioma cells U87-V, U87-M, snb19-V and snb19-M in the logarithmic growth phase are prepared into single-cell suspensions respectively at a concentration of $1.5 \times 10^6$/ml. 1ml single-cell suspension is cultured in a 150mm culture dish for 3 days and digested with trypsin. The treated cells are harvested and washed twice with pre-cooling PBS. 2mL of pre-cooling 60% methanol aqueous solution is added to carry out extraction. Cells are broken up by sonication 5 times for 3s, with 3s interval between each sonication, operated on an ice bath. Centrifugation is carried out, the supernatant is collected into a sample vial, and the precipitate is extracted with 1ml of 60% methanol. The supernatants are combined, freeze-dried, and left overnight. The resulted is redissolved in 300ul pre-cooling 60% methanol aqueous solution, vortex sonicated, filtered with a 0.22 $\mu$m membrane, and detected. The results are shown in Figure 4: In snb19 cells, the intracellular methionine level in group M is decreased 19.9 times compared with group V. However, in U87 cells, the group M is decreased 3.15 times compared with the group V. These results indicate that MEGL overexpression virus has successfully infected the cells and reduced the intracellular methionine content.

**4. Detection of effects of MEGL overexpression virus on the proliferation inhibition of glioma cells by CCK8 method**

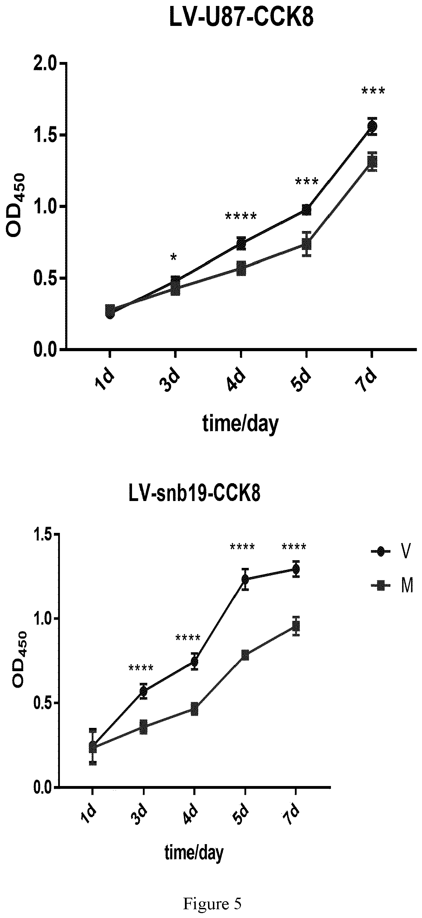

Single-cell suspensions are prepared with virus infected glioma cells U87-V, U87-M, snb19-V and snb19-M in the logarithmic growth phase, and seeded in a 96-well plate, with 100$\mu$L cell suspension (containing $1.5 \times 10^3$ cells) per well, 5 duplicates for each group; The proliferation of cells is detected on the 1st, 3rd, 4th, 5th and 7th days respectively as follows: 10ul CCK-8 solution is added to each well without generating bubbles; The cells are incubated in an incubator at 37 °C with 5% $CO_2$ for 1h, then the culture plate is taken out, and the absorbance of the cells at 450 nm is measured with a microplate reader. Results shown in Figure 5 indicate MEGL overexpression virus has significantly inhibited the proliferation of glioma cells.

**5. Real time PCR detection of decrease in EZH2 gene expression in tumor cells by MEGL overexpression virus**

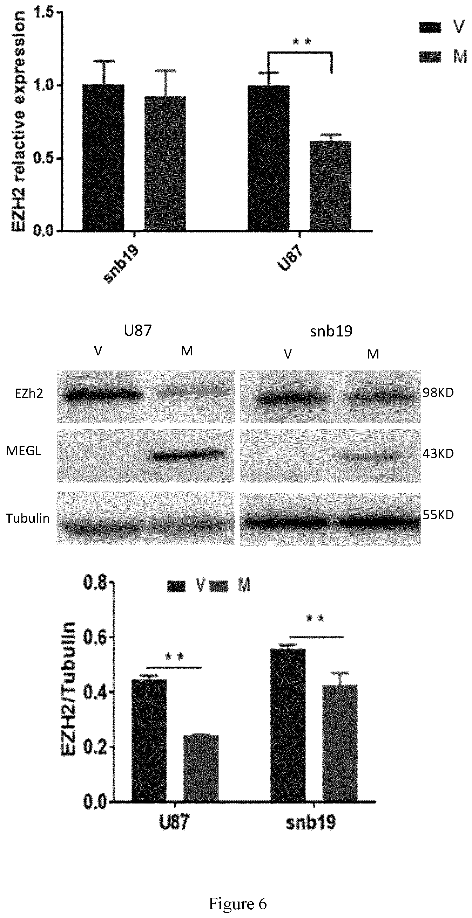

Total RNA is extracted from well-grown cells after virus infection by using Trizol method. 1$\mu$g RNA template is reverse transcribed into cDNA in vitro. Using cDNA as template, real time PCR is carried out at following reaction condition: 50 °C for 2 min; 95 °C for 10 min; 95 °C for 15 sec, 60 °C for 1 min; 72 °C for 1 min (40 cycles in total), and finally a product dissolution curve is measured, and calculation is carried out using $2^{-\triangle\triangle Ct}$ method. Results shown in Figure 6A show MEGL overexpression virus has reduced the expression of EZH2 gene in cells. As shown in Figure 6B, Western Blot results further confirms that MEGL overexpression virus has reduced the expression of EZH2 protein in cells.
The above in vitro experiments further show that the virus mediated methioninase system in present invention can inhibit the proliferation of tumors by inhibiting EZH2, and has good anti-tumor effects.

**Claims**

**1.** A viral vector, **characterized in that**, an exogenous MEGL gene is inserted therein.

2.  The viral vector according to claim 1, **characterized in that**, the exogenous MEGL gene is a methionine γ- lyase gene.

3.  The viral vector according to claim 1 or 2, **characterized in that**, the vector uses EF1A promoter.

4.  The viral vector according to any one of claims 1-3, **characterized in that** the vector carries a mcherry fluorescent protein.

5.  The viral vector according to any one of claims 1-4, **characterized in that** the exogenous MEGL gene consists of a sequence of SEQ ID No. 1.

6.  The viral vector according to any one of claims 1-5, **characterized in that** the vector is constructed by following steps of: subcloning the MEGL gene into a plasmid to obtain a MEGL expression plasmid; transfecting the MEGL expression plasmid and a helper plasmid into 293T cells; and collecting a supernatant, concentrating and purifying to obtain the viral vector.

7.  The viral vector according to claim 6, **characterized in that** the MEGL expression plasmid and the helper plasmid are co-transfected into the 293T cells, and the helper plasmid is a viral packaging helper plasmid.

8.  The viral vector according to claim 6 or 7, **characterized in that** the MEGL expression plasmid carries a mCherry red fluorescent protein.

9.  The viral vector according to any one of claims 6-8, **characterized in that** the vector is constructed by following steps of:

    (a) constructing an entry vector using BP reaction, comprising: mixing a Gateway expression vector having a target gene attB1- MEGL - attB2 sequence with a donor vector having attP 1- ccdB - attP2 sequence; adding a BP Clonase enzyme mixture containing Int and IHF, keeping at 25 °C for 1h, and treating with a protease K at 37 °C for 10min to generate an entry vector having target gene MEGL and an expression vector having a suicide gene; transforming the entry vector into Escherichia coli Stbl3, and idendifying positive clones and performing sequencing validation;
    (b) constructing a destination vector having two recombination sites attR1 and attR2 downstream of an expression regulatory element thereof, each of the recombination sites being 125 bp in length; and having a ccdB suicide gene between the attR1 and attR2; and
    (c) constructing a final expression vector via LR reaction, comprising: mixing the entry vector and the destination vector, adding a LR Clonase enzyme mixture containing recombinant factors including Int, IHF and Xis, keeping at 25 °C overnight, performing transformation by treating with a proteinase K at 37 °C for 10 minutes to generate a fusion plasmid, the attL1 sequence and the attR1 sequence being recombined, the fusion plasmid being decomposed into two new plasmids, obtaining a final expression vector of destination vector having the target gene; transforming the final expression vector into Escherichia coli Stbl3, and idendifying positive clone plasmid and performing sequencing validation.

10. The viral vector according to any one of claims 1-9, **characterized in that** the viral vector is a lentivirus vector.

11. A method for constructing the viral vector according to any one of claims 1-10, **characterized by** comprising: subcloning the MEGL gene into a plasmid to obtain a MEGL expression plasmid, transfecting the MEGL expression plasmid and a helper plasmid into 293T cells; collecting the supernatant, concentrating and purifying to obtain the target virus.

12. The method according to claim 11, **characterized in that**, the MEGL expression plasmid and the helper plasmid are co-transfected into the 293T cells, and the helper plasmid is viral packaging helper plasmid,

13. The method according to claim 11 or 12, **characterized in that** the MEGL expression plasmid carries a mCherry red fluorescent protein.

14. The method according to any one of claims 11-13, **characterized by** comprising

    (a) constructing an entry vector using BP reaction, comprising: mixing a Gateway expression vector having a target gene attB1- MEGL - attB2 sequence with a donor vector having attP 1- ccdB - attP2 sequence; adding a BP Clonase enzyme mixture containing Int and IHF, keeping at 25 °C for 1h, and treating with a protease K at 37 °C for 10min to generate an entry vector having target gene MEGL and an expression vector having a suicide gene; transforming the entry vector into Escherichia coli Stbl3, and idendifying positive clones and performing sequencing validation;

(b) constructing a destination vector having two recombination sites attR1 and attR2 downstream of an expression regulatory element thereof, each of the recombination sites being 125 bp in length; and having a ccdB suicide gene between the attR1 and attR2; and

(c) constructing a final expression vector via LR reaction, comprising: mixing the entry vector and the destination vector, adding a LR Clonase enzyme mixture containing recombinant factors including Int, IHF and Xis, keeping at 25 °C overnight, performing transformation by treating with a proteinase K at 37 °C for 10 minutes to generate a fusion plasmid, the attL1 sequence and the attR1 sequence being recombined, the fusion plasmid being decomposed into two new plasmids, obtaining a final expression vector of destination vector having the target gene; transforming the final expression vector into Escherichia coli Stbl3, and idendifying positive clone plasmid and performing sequencing validation

15. Use of the viral vector according to any one of claims 1-10 or the viral vector constructed by the method according to any one of claims 11-14 in the manufacture of a medicament for treating malignant tumors.

16. The use according to claim 15, **characterized in that** the medicament directly kills tumor cells.

17. The use according to 15 or 16, **characterized in that** the malignant tumor is glioma.

18. Use of the viral vector according to any one of claims 1-10 or the viral vector constructed by the method according to any one of claims 11-14 in the manufacture of histone methyltransferase EZH2 inhibitor.

19. A method for treating malignant tumors, comprising administering the viral vector according to any one of claims 1-10 or the viral vector constructed by the method according to any one of claims 11-14 to subjects in need.

20. The method according to claim 19, **characterized in that** the malignant tumor is glioma.

# Vector Map

Figure 1

10×

**U87**

**snb19**

Bright field

Fluorescence

Combination

Figure 2

Figure 3

## U87-Met

| | V | M |
|---|---|---|
| 3d | 2.23493512e+008 | 7.0932328e+007 |

## snb19-Met

| | V | M |
|---|---|---|
| 3d | 8.9317652e+007 | 4482134.00 |

Figure 4

Figure 5

Figure 6

## INTERNATIONAL SEARCH REPORT

| International application No. |
| --- |
| **PCT/CN2020/105381** |

| A. | CLASSIFICATION OF SUBJECT MATTER |
| --- | --- |

C12N 15/60(2006.01)i; A61K 35/18(2015.01)i

According to International Patent Classification (IPC) or to both national classification and IPC

| B. | FIELDS SEARCHED |
| --- | --- |

Minimum documentation searched (classification system followed by classification symbols)

C12N;A61K

Documentation searched other than minimum documentation to the extent that such documents are included in the fields searched

Electronic data base consulted during the international search (name of data base and, where practicable, search terms used)

VEN, CNABS, PubMed, CNKI, ISI Web of Knowledge, 病毒载体, 慢病毒, 腺病毒, 肿瘤, 甲硫氨酸, 蛋氨酸, 甲硫氨酸γ-裂解酶, 甲硫氨酸酶, L-methionine γ-lyase, MGL, METase, MEGL, Adenovirus Vector, lentiviral vector

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2019183294 A1 (RUBIUS THERAPEUTICS INC.) 26 September 2019 (2019-09-26) <br> pages 51 and 170-172, embodiment 2, and table 2 | 1-14 |
| Y | WO 2019183294 A1 (RUBIUS THERAPEUTICS INC.) 26 September 2019 (2019-09-26) <br> pages 51 and 170-172, embodiment 2, and table 2 | 15-20 |
| X | 任晓丽等 (REN, Xiaoli et al.). "蛋氨酸酶基因重组腺病毒载体构建 (Construction of L-Methionine γ-Lyase Gene Recombinant Adenovirus Vector)" <br> 昆明医科大学学报 (Journal of Kunming Medical University), <br> Vol. 34, No. 6, 31 December 2013 (2013-12-31), <br> introduction, 1 materials and methods, 3 discussion | 1, 2, 15-20 |
| Y | 任晓丽等 (REN, Xiaoli et al.). "蛋氨酸酶基因重组腺病毒载体构建 (Construction of L-Methionine γ-Lyase Gene Recombinant Adenovirus Vector)" <br> 昆明医科大学学报 (Journal of Kunming Medical University), <br> Vol. 34, No. 6, 31 December 2013 (2013-12-31), <br> introduction, 1 materials and methods, 3 discussion | 15-20 |
| A | CN 101955959 A (CHANGCHUN UNIVERSITY OF SCIENCE AND TECHNOLOGY) 26 January 2011 (2011-01-26) <br> entire document | 1-20 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 March 2021** | **22 March 2021** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088** <br> **China** | |
| Facsimile No. **(86-10)62019451** | Telephone No. |

Form PCT/ISA/210 (second sheet) (January 2015)

## INTERNATIONAL SEARCH REPORT

| | International application No. |
|---|---|
| | **PCT/CN2020/105381** |

**C. DOCUMENTS CONSIDERED TO BE RELEVANT**

| Category* | Citation of document, with indication, where appropriate, of the relevant passages | Relevant to claim No. |
|---|---|---|
| A | CN 104928310 A (HUBEI UNIVERSITY OF TECHNOLOGY) 23 September 2015 (2015-09-23)<br>entire document | 1-20 |
| A | 龚娇等 (GONG, Jiao et al.). "蛋氨酸依赖性对肿瘤生长抑制的研究进展 (Research Progress of Methionine Dependence Inhibition of Tumor Growth)"<br>现代肿瘤医学 (Journal of Modern Oncology),<br>Vol. 25, No. 3, 28 February 2017 (2017-02-28),<br>entire document | 1-20 |
| A | 胡海艳等 (HU, Haiyan et al.). "甲硫氨酸γ-裂解酶基因在大肠杆菌中的高效表达和活性 (High Expression and Activity of an L-methionine γ-lyase Gene in Escherichia Coli)"<br>微生物学通报 (Microbiology China), Vol. 12, No. 46, 20 December 2019 (2019-12-20),<br>entire document | 1-20 |
| A | Tan, Y.Y. et al. "Overexpression and Large-Scale Production of Recombinant L-Methionine-a-deamino-γ-mercaptomethane-lyase for Novel Anticancer Therapy"<br>Protein Expression and Purification, 31 December 1997 (1997-12-31),<br>entire document | 1-20 |
| A | 夏立亮等 (XIA, Liliang et al.). "L-蛋氨酸γ-裂解酶的研究进展 (Research Progress on L-Methionineγ-Lyase)"<br>生物技术通报 (Biotechnology Bulletin), No. 10, 31 December 2009 (2009-12-31),<br>entire document | 1-20 |

Form PCT/ISA/210 (second sheet) (January 2015)

**INTERNATIONAL SEARCH REPORT**

International application No.

**PCT/CN2020/105381**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

       ☑ in the form of an Annex C/ST.25 text file.

       ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

       ☐ in the form of an Annex C/ST.25 text file (Rule 13*ter*.1(a)).

       ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.

**PCT/CN2020/105381**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **19-20**
because they relate to subject matter not required to be searched by this Authority, namely:

    [1]    Claims 19 and 20 relate to methods of treatment of diseases, and are a subject matter that therefore does not warrant an international search according to the criteria set out in PCT Rule 39.1(iv). This report is made on the basis of a search for "the use of the described viral vectors in the preparation of drugs for the treatment of malignant tumors".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2020/105381**

| Patent document cited in search report | | | Publication date (day/month/year) | Patent family member(s) | | | Publication date (day/month/year) |
|---|---|---|---|---|---|---|---|
| WO | 2019183294 | A1 | 26 September 2019 | US | 2019309271 | A1 | 10 October 2019 |
| CN | 101955959 | A | 26 January 2011 | | None | | |
| CN | 104928310 | A | 23 September 2015 | US | 2017260518 | A1 | 14 September 2017 |
| | | | | WO | 2016197561 | A1 | 15 December 2016 |
| | | | | CN | 104928310 | B | 11 May 2018 |
| | | | | US | 10308925 | B2 | 04 June 2019 |

Form PCT/ISA/210 (patent family annex) (January 2015)